# EUROPÄISCHE PATENTSCHRIFT

(11) **EP 1 693 357 B1**
(45) Veröffentlichungstag und Bekanntmachung des Hinweises auf die Patenterteilung: **16.01.2008**
(21) Anmeldenummer: 05003760.5
(22) Anmeldetag: 22.02.2005
(51) Int. Cl.: C07C 6/06, B01J 8/02

(54) **Verfahren und Anlage zur Durchführung einer ringöffnenden Kreuzmetathesereaktion zwischen cyclischen und acyclischen Olefinen**
Process and apparatus for carying out a ring opening cross-metathesis reaction between cyclic and acyclic olefins
Procédé et installation pour la mise en oeuvre d'une réaction métathèse croisée à ouverture du cycle entre des oléfines cycliques et acycliques

(43) Veröffentlichungstag der Anmeldung: 23.08.2006
(73) Patentinhaber: Miltitz Aromatics GmbH, 06766 Wolfen (DE)
(72) Erfinder: Müller, Peter, D-04229 Leipzig (DE); Braband, Jürgen, D-04275 Leipzig (DE); Doerfelt, Stephan, D-08371 Glauchau (DE); Otto, Andreas, D-06800 Jessnitz (DE)
(74) Vertreter: Tragsdorf, Bodo

(56) Entgegenhaltungen:
- WO-A-00/07967
- US-A- 4 668 836

## Beschreibung

Die Erfindung bezieht sich auf ein Verfahren zur Durchführung einer ringöffnenden Kreuzmetathesereaktion zwischen einem flüssigen cyclischen Olefin und einem acyclischen Olefin als Reaktionsgas an einem metathetisch aktiven Katalysatorsystem als Festbett sowie auf eine zur Durchführung des Verfahrens geeignete Anlage.

Unter Metathese versteht man die wechselseitige Umalkylidenlerung von Alkenen. Je nach Art der Ausgangskomponenten und der Reaktionsführung unterscheidet man die Metathese in folgende Reaktionstypen:

Die Ringschlussmetathese (RCM) als Cyclisierung eines Dlens zu einem Cycloolefin, bei der aus einem Edukt zwei Produktmoleküle entstehen und als Nebenprodukt ein aus der Reaktionsmischung entweichendes Olefin.

Die Ringöffnungsmetathese (ROM) ist die Rückreaktion der RCM, z. B. als selektive Ringöffnung von cyclischen Olefinen mit acylischen Olefinen, wobei die freiwerdende Ringspannung die Triebkraft der Reaktion bildet. Die Reaktion erfolgt z.B. unter Beisein eines Überschusses an acyclischem Olefin.

Durch eine Ringöffnungsmetathese-Polymerisation (ROMP) werden ausgehend von cyclischen Olefinen ungesättigte Oligomere oder Polymere erhalten. Der Unterschied zur ROM besteht darin, dass als Edukt kein zweites Olefin vorhanden ist. Dieser Reaktionstyp hat in der Praxis bereits eine breite Anwendung gefunden, im Gegensatz zur Acyclischen Dien-Metathese-Polymerisation (ADMET) als Polymerisation von acyclischen α, ω-Dienen unter Freisetzung von Ethen.

Ein weiterer Reaktionstyp ist die Kreuzmetathese (CM), die eine Umsetzung von zwei unterschiedlichen Olefinen beschreibt. Ist einer der Reaktionspartner Ethen, so handelt es sich um eine Ethenolyse.

Bei der ringöffnenden Kreuzmetathese von cyclischen Olefinen mit acylischen Olefinen besteht die Gefahr, dass außer dem Zielprodukt, durch eine Selbstmetathese des Cycloolefins, Cyclooligomere und -polymere entstehen.

Für die Selektivität der Reaktionen sind daher die Auswahl geeigneter Cycloolefine hinsichtlich leichter Ringöffnung, Katalysatoren und Reaktionsbedingungen von wesentlicher Bedeutung.

Aus der EP 0 182 333 B1 und EP 0343 437 B1 sind Verfahren zur Durchführung einer ringöffnenden Metathesereaktion von cyclischen Olefinen an einem metathetisch aktiven Katalysatorsystem, bestehend aus einem Trägerkatalysator und einem Cokatalysator bekannt. Die Reaktion erfolgt in einem mit Katalysator gefülltem

Rohrreaktor in vertikaler Anordnung (als Festbett), wobei die Reaktionslösung diesen durchströmt. Um die Bildung an höhermolekularen Verbindungen möglichst gering zu halten, werden die Cycloolefine mittels geeigneter Lösungsmittel verdünnt, auf Konzentrationen von < 0,05 mol/l. Als Lösungsmittel kommen im allgemeinen Kohlenwasserstoffe (Pentan, Hexan, Toluen, Xylen) oder chlorierte Kohlenwasserstoffe (Dichlormethan, 1,2-Dlchlorethan, Halogenbenzole) in Frage.

Aus der EP 0 522 067 B1 ist eine Metathesereaktion zwischen einem cyclischen Olefin (Cycloocten) und einem acyclischen Olefin (Ethen) als Reaktionsgas bekannt. Die Reaktion erfolgt in einem Laborautoklaven bei einem Ethendruck von 8 bar an einem Katalysator auf Basis Methylrhenlumtrioxid (Katalysatorträger SiO₂/Al₂O₃). Das cyclische Olefin wurde in einer Konzentration von 0,05 mol/l eingesetzt. Wie in dieser Druckschrift angegeben, soll die Ausbeute an Zielprodukt (1,9-Decadien) bei 91% liegen.

Der Einsatz großer Mengen an Lösungsmittel (Konzentrationen von < 0,1 mol/l) ist mit einer Vielzahl an Nachteilen verbunden. Hohe Verdünnungen erfordern großvolumige Reaktionsbehälter und einen entsprechenden Dosieraufwand. Das Lösungsmittel muss nachträglich wieder aus dem Reaktionsgemisch entfernt werden. Der Umgang mit großen Mengen an Lösungsmittel stellt eine Gefahr für die Umwelt dar und führt zu einer Erhöhung der Kosten.

Auch bei hohen Verdünnungen laufen noch eine Vielzahl von Konkurrenzreaktionen am metathetisch aktiven Katalysator ab, da jedes neu entstehende Produkt über olefinische Bindungen verfügt, die wieder mit sich selbst (Selbstmetathese), den Ausgangsprodukten oder anderen bereits entstandenen olefinischen Produkten reagieren können.

Die unerwünschte Bildung von Oligomeren und Polymeren führt beim Einsatz von Festbettkatalysatoren zu einer Deaktivierung des Katalysators, durch Verstopfung der Katalysatorporen oder zum "Verkleben" der aktiven Katalysatoroberfläche (sogenanntes "Fouling"). Dies hat zur Folge, dass die Reaktivität des Katalysators und dessen Standzelt erheblich verringert werden. Angaben in der Literatur zu erzielten Ausbeuten von *>* 90%, die im Rahmen von Laborversuchen gaschromatographisch ermittelt wurden, sind für eine großtechnische Umsetzung in der Praxis unrealistisch. Untersuchungen haben ergeben, dass die Zielprodukte noch erhebliche Anteile an höhermolekularen Verbindungen enthalten, die bei der Bestimmung der Ausbeute mittels Gaschromatographie nicht mit erfasst wurden, so dass die tatsächlichen Ausbeuten deutlich unter den ermittelten Werten liegen.

Für die Wirtschaftlichkeit der Verfahrensweise, insbesondere zur Erzielung hoher Ausbeuten, ist es daher wünschenswert, sowohl die Konzentration an Edukt möglichst hoch zu halten als auch die Bildung von längerkettigen Polymeren bereits während der Reaktion weitestgehend zu unterbinden.

Der Erfindung liegt die Aufgabe zugrunde, ein Verfahren zur Durchführung einer ringöffnenden Kreuzmetathesereaktion zwischen einem flüssigen cyclischen Olefin und einem acyclischen Olefin als Reaktionsgas an einem metathetisch aktiven Katalysatorsystem als Festbett in einem Reaktor zu schaffen, das sich durch eine verbesserte Wirtschaftlichkeit auszeichnet, insbesondere zu höheren Ausbeuten an niedermolekularem Zielprodukt führt und bei dem die Bildung höhermolekularer Verbindungen weitestgehend verhindert wird. Ferner soll eine zur Durchführung des Verfahrens geeignete Anlage geschaffen werden.

Erfindungsgemäß wird die Aufgabe durch die im Anspruch 1 angegebenen Merkmale gelöst. Vorteilhafte Ausgestaltungen und Weiterbildungen der Verfahrensweise sind Gegenstand der Ansprüche 2 bis 12. Eine geeignete Anlage zur Durchführung des Verfahrens im technischen Maßstab ist im Anspruch 13 angegeben. Vorteilhafte Ausgestaltungen dieser Anlage sind Gegenstand der Ansprüche 14 bis 17.

Während der einzelnen Reaktionszyklen, wird in das im Kreislauf gefahrene Reaktionsgemisch zusätzlich, außer dem bereits in gelöster Form vorliegenden Reaktionsgas, noch Reaktionsgas feinblasig dispergiert. Die dispergierte Menge an Reaktionsgas sollte mindestens 25 Vol.-%, bezogen auf das im Kreislauf vorhandene Flüssigkeitsvolumen, betragen. Die durch die Dispergierung gebildeten Feinblasen besitzen einen Durchmesser von 0,1 bis 1 mm. Durch die Dispergierung kommt es zu einer Verwirbelung des Reaktionsgemisches, wobei dieses als nahezu homogene Gas-Flüssigkeitsmischung umgewälzt wird. Die Feinblasen sind im Reaktionsgemisch homogen verteilt. Zur Umsetzung wird im Lösungsmittel gelöstes Reaktionsgas verbraucht. Da am Festbettkatalysator ständig eine ausreichende Menge an gelöstem und dispergiertem Reaktionsgas zur Verfügung steht, erfolgt die Umsetzung bevorzugt zu niedermolekularen Verbindungen bzw. vorhandene höhermolekulare Verbindungen werden verstärkt abgebaut. Außer dem Zielprodukt entstehen als Nebenprodukt hauptsächlich Oligomere mit bis zu drei Cycloolefineinheiten, die nach separater Abtrennung aus der Reaktionslösung wieder in den Kreislauf zurückgeführt werden. Dies führt zu einer deutlichen Verbesserung der Wirtschaftlichkeit des Verfahrens.

Der Anteil an niederen Oligomeren (Trimere, Tetramere) in der Reaktionslösung wird im Vergleich zu einer Verfahrensweise mit ausschließlich bis zur Sättigungskonzentration gelöstem Reaktionsgas deutlich erhöht. Durch die erfindungsgemäße Dispergierung von Reaktionsgas kann somit gezielt Einfluss auf die Selektivität der Metathesereaktion genommen werden.

Es wird vermutet, dass bedingt durch das ständige Nachlösen von dispergiertem Reaktionsgas, während der Umsetzung am Katalysator acyclisches Olefin in ausreichender Menge vorliegt und dadurch statistisch bevorzugt an den reaktiven Zentren des Katalysators angelagert wird. Dadurch wird eine sehr günstige Oligomerenverteilung erzielt, wobei als Nebenprodukte vorwiegend Verbindungen mit maximal bis zu drei Olefineinheiten anfallen.

Auch bei einer alleinigen Erhöhung des Druckes (bis zu 20 bar) des Reaktionsgases, der zu einer höheren Konzentration von gelöstem acyclischen Olefin (Reaktionsgas) im Lösungsmittel führt, konnte im Vergleich zur Dispergierung von Reaktionsgas keine wesentliche Verbesserung des Reaktionsverlaufes festgestellt werden. Dieser Maßnahme sind auch aus anlagentechnischer Sicht Grenzen gesetzt. Erst durch eine zusätzliche Dispergierung von Reaktionsgas konnte völlig überraschend der Anteil an höhermolekularen Verbindungen in der Reaktionslösung verringert und die Ausbeute an Zielprodukt erhöht werden. Bedingt durch den geringeren Anteil an höhermolekularen Verbindungen kommt es auch zu einer Verlängerung der Standzeiten des Katalysatorsystems.

Es hat sich als vorteilhaft herausgestellt, wenn die Reaktion in einem Schleifenreaktor mit integriertem Festbettkatalysator und einem In die Kreislaufschleife eingebundenem Ausdehnungsgefäß erfolgt. Zur Dispergierung des Reaktionsgases wird vorzugsweise eine Strahlpumpe eingesetzt, die am Kopf des Ausdehnungsgefäßes angeordnet ist, und das zu dispergierende Reaktionsgas sowohl aus dem Ausdehnungsgefäß ansaugt als auch in das Reaktionsgemisch, unter intensiver Verwirbelung, einträgt.

In Versuchen hat sich herausgestellt, dass die Reaktion auch bei einer um ein Vielfaches geringeren Verdünnung des cyclischen Olefins erfolgreich durchgeführt werden kann. Das cyclische Olefin kann in Konzentrationen von 0,1 bis 5 Mol/l eingesetzt werden. Für eine wirtschaftliche Betriebsweise im technischen Maßstab ist dies von großer Bedeutung. Die erforderlichen Einsatzmengen an Lösungsmittel beeinflussen die Verfahrenskosten erheblich.

Im verfahrenstechnischen Ablauf für eine technische Umsetzung in einem Schleifenreaktor wird vor der Zuführung des cyclischen Olefins das Katalysatorsystem unter inerten Bedingungen in die Festbetteinrichtung eingetragen, diese anschließend mit einem metathetisch inerten Lösungsmittel aufgefüllt und die Festbetteinrichtung dicht verschlossen. Während des geschlossenen Zustandes der Festbetteinrichtung wird in den Schleifenreaktor die erforderlichen Menge an metathetisch inertem Lösungsmittel gegeben, Inertgas eingeleitet und danach Inertgas durch unter Überdruck zugeführtes Reaktionsgas ausgetauscht.

Nach erfolgtem Austausch von inertgas durch Reaktionsgas wird die Festbetteinrichtung geöffnet und das Gemisch Lösungsmittel/Reaktionsgas unter Zuführung von Rektionsgas solange im Kreislauf gefahren, bis keine Gasaufnahme mehr erfolgt. Als Lösungsmittel für das Reaktionsgas wird vorzugsweise Toluen eingesetzt. Anschließend erfolgen mehrere Reaktionszyklen, durch Zudosierung von mit Lösungsmittel, vorzugsweise Toluen, verdünntem cyclischem Olefin, vorzugsweise In Konzentrationen von 0,1 bis 5 Mol/l, und dem Eintrag von dispergiertem Reaktionsgas (acyclisches Olefin) über die Strahlpumpe. Das Reaktionsgemisch wird unter Zuführung von Reaktionsgas durch Dispergierung solange im Kreislauf gefahren, bis keine Aufnahme an Reaktionsgas mehr erfolgt. Dieser Zeitpunkt Ist erreicht, wenn der Volumenstrom an Reaktionsgas eine bestimmte Untergrenze unterschreitet, die bei Ethen 0,2 Normliter und bei isobuten 0,3 Normliter beträgt.

Ein Reaktionszyklus ist beendet, wenn keine Gasaufnahme mehr feststellbar ist.

Nach Beendigung eines jeden Reaktionszyklus wird eine Teilmenge an Reaktionslösung ausgekreist und destillativ in Lösungsmittel, Zielprodukt, Oligomerengemisch und gegebenenfalls Destillationsrückstand getrennt. Nach dem Auskreisen einer Teilmenge an Reaktionslösung beginnt ein neuer Reaktionszyklus mit der Zudosierung von cyclischem Olefin und der Nachdosierung von Reaktionsgas, wie vorstehend erwähnt, und erforderlichenfalls durch Zuführung von Lösungsmittel für das Reaktionsgas.

Während des Verfahrensablaufes wird die Aktivität des Katalysatorsystems durch gaschromatografische Untersuchungen der ausgekreisten Reaktionslösungen überwacht, und bei nicht mehr ausreichend vorhandener Aktivität des Katalysatorsystems, die Metathesereaktion abgebrochen und das Katalysatorsystem ausgetauscht und die Verfahrensweise wieder neu begonnen. Es besteht auch die Möglichkeit, in den Schleifenreaktor eine oder mehrere Festbettkatalysatoreinrichtungen einzubinden, sodass die Verfahrensweise wechselseitig über die jeweils zugeschaltete Festbettkatalysatoreinrichtung fortgesetzt wird.

Vorzugsweise wird in den einzelnen Reaktionszyklen das zuzuführende cyclische Olefin vollständig oder teilweise durch aus der ausgekreisten Reaktionslösung zurückgewonnenes Oligomerengemisch ersetzt. Zurückgewonnenes Lösungsmittel und gegebenenfalls unumgesetztes acyclisches Olefin können ebenfalls wieder eingesetzt werden. Als Lösungsmittel kommen die an sich bekannten Mittel, wie z.B. Toluen, Xylen, Pentan, Hexan oder Heptan in Frage.

Verfahrenstechnisch ist es sinnvoll, wenn für das Reaktionsgas (acyclische Komponente) und das cycllsche Olefin identische Lösungsmittel verwendet werden. Als cyclische Olefine können folgende Verbindungen eingesetzt werden: Cyclopenten, Cyclohepten, Cycloocten, 1,5-Cycloactadien, 1,5-Dimethyl-1,5-cyclooctadien. Cyclodecen, Cyclododecen, 1,9 Cyclohexadecadien, 1,9,17 Tetracosa- trien, Alpha-Pinen, Caren, Limonen, Norbornen oder Norbornadien.

Außerdem sind auch andere homologe Verbindungen dieser Reihe geeignet.

Wie bereits erwähnt, wird das cyclische Olefin bevorzugt in Konzentrationen von 0,1 bis 5 Mol/l eingesetzt. Die bekannten Verfahren erfordern Einsatzkonzentrationen von < 0.05 mol/l. Im Vergleich zu den bekannten Verfahren kann die Reaktion mit einer um das Vielfache geringeren Menge an Lösungsmittel durchgeführt werden. Da in der Praxis der Umgang mit größeren Mengen an Lösungsmittel problematisch ist, und die Abtrennung des Lösungsmittels aus der Reaktionslösung mit einem erheblichen Aufwand verbunden ist, wirken sich höhere Einsatzkonzentrationen vorteilhaft auf die Verfahrensökonomie aus.

Als acylisches Olefin werden insbesondere solche Olefine eingesetzt, die bei Raumtemperatur als Gase vorliegen, wie Ethen, Propen, Buten-1, Buten-2 oder Isobuten. Außerdem sind auch Olefine geeignet, die durch Verdampfung In den gasförmigen Zustand überführt werden können, wie 1-Penten, 2-Penten, 2-Methyl-2-buten (Isoamylen), 1-Hexen, 2-Hexen, 3-Hexen, 2-Methyl-1-penten, 3-Methyl-1-penten, 4-Methyl-1-penten (Isohexen), Neohexen, 2-Methyl-2-penten, 3-Methyl-2-penten und 4-Methyl-2-penten.

Das eingesetzte Katalysatorsystem besteht aus einem aktivierten Trägerkatalysator, vorzugsweise auf Basis Re₂O₇-B₂O₃/Al₂O₃, und einem geeigneten Co-Katalysator, wie Me₄Sn oder anderen tetraalkylierten Zinnverbindungen.

Prinzipiell können alle am Markt verfügbaren Metathesekatalysatoren für Festbettelnrichtungen verwendet werden.

Aus vorgenannten Edukten können nach der erfindungsgemäßen Verfahrensweise als Zielprodukte Diene, z. B. 1,9 Decadien oder 7-Methyl-1,6-octadien, hergestellt werden. Das vorgeschlagene Verfahren und die dazugehörige Anlage ermöglichen die Durchführung der Reaktion mit gasförmigen acyclischen Olefinen bei niedrigen Reaktionstemperaturen sowie niedrigen Gasdrücken, wobei gute Raum-Zeit-Ausbeuten und hohe Selektivitäten bezüglich der primären Metatheseprodukte erreicht werden. Ohne zusätzliche Reinigungsstufe werden vergleichsweise erheblich längere Standzeiten des Katalysatorsystems erzielt.

Die Erfindung soll nachstehend an mehreren Beispielen erläutert werden. In der zugehörigen Zeichnung ist der Aufbau einer Anlage zur Durchführung des Verfahrens in vereinfachter schematischer Darstellung gezeigt.

Die Anlage besteht aus einem Schleifenreaktor SR mit einer Kreislaufleitung L, in die eine absperrbare Festbettkatalysatoreinrichtung 1, ein Ausdehnungsgefäß 2, eine Strahlpumpe 3 und eine Kreislaufpumpe 4 (frequenzgesteuerte Mehrphasenpumpe) eingebunden sind. Das Ausdehnungsgefäß 2 ist, in Strömungsrichtung gesehen, vor der Kreislaufpumpe 4 und die Festbetteinrichtung 1 nach der Kreislaufpumpe 4 angeordnet. Die in vertikaler Anordnung vorgesehene Festbetteinrichtung 1 liegt In Strömungsrichtung hinter der Kreislaufpumpe 4 und vor der Strahlpumpe 3. Die Strahlpumpe 3 Ist in den Abschnitt der Kreislaufleitung L eingebunden, der sich unmittelbar oberhalb des Ausdehnungsgefäßes 2 befindet. Der Saugstutzen 3a der Strahlpumpe 3 Ist über eine Leitung 7 mit dem zugehörigen Vorratsbehälter für das Reaktionsgas bzw. Inertgas verbunden. In die Leitung 7 ist ein Absperrventil 10 eingebunden. Zwischen dem Absperrventil 10 und dem Sauganschluss 3a ist in die Leitung 7 noch eine Abzweigleitung 5 eingebunden (Einbindungsstelle 9), die in das Ausdehnungsgefäß 2 mündet. Wenn das Absperrventil 10 geschlossen ist, so wird über die Leitung 5 Reaktionsgas aus dem Ausdehnungsgefäß 2 angesaugt. Der Diffusor 3b der Strahlpumpe 3 ragt in das Ausdehnungsgefäß 2. Der Flüssigkeitsanschluss 3c der Strahlpumpe 3 ist mit der Kreislaufleitung L verbunden. Vor der Strahlpumpe 3 ist in die Kreislaufleitung L eine absperrbare Zuführungsleitung 6 eingebunden, durch die cyclisches Olefin oder Lösungsmittel zugeführt werden.

Es ist auch möglich, die Leitung für die Zuführung von Reaktionsgas bzw. Inertgas aus einem Vorratsbehälter vor der Strahlpumpe 3 In die Kreislaufleitung L einzubinden. In diesem Fall ist dann der Sauganschluss 3a der Strahlpumpe 3 nur mit der Leitung 5 verbunden.

Nach der Festbetteinrichtung 1, hinter dem Absperrventil 12, ist in die Kreislaufleitung

L eine absperrbare Leitung 8 zur Auskreisung der Reaktionslösung eingebunden. Die Kreislaufleitung L ist in dem Abschnitt zwischen der Kreislaufpumpe 4 und der Festbettelnrichtung 1 mit einem Absperrventil 11 ausgerüstet.

Die Kreislaufleitung des Schleifenreaktors besitzt Verwirbelungsabschnitte, die für eine weitere Durchmischung des im Kreislauf gefahrenen Reaktionsgemisches sorgen.

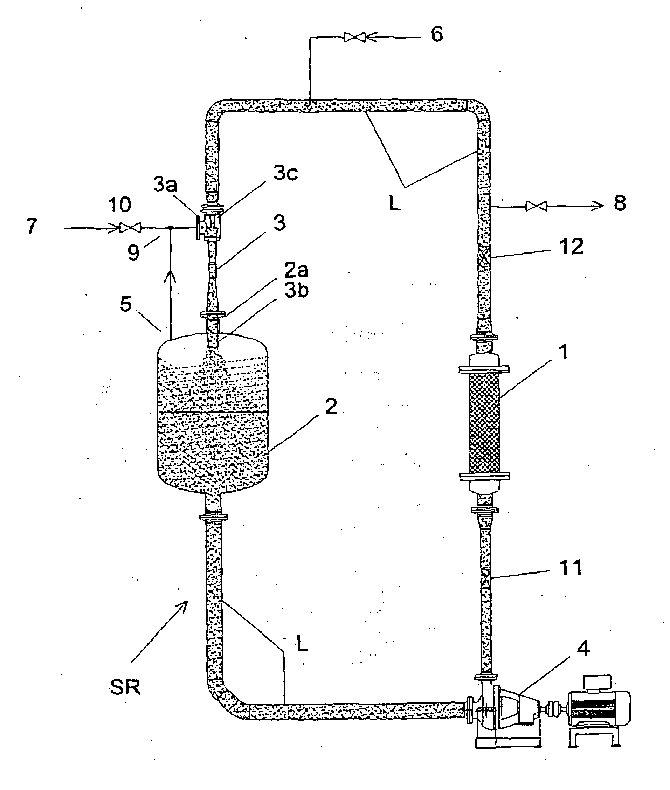

Der Schleifenreaktor ist mit den erforderlichen Messeinrichtungen zur Überwachung der Verfahrensparameter, wie Druck und Temperatur, sowie mit einer Durchflussmesseinrichtung ausgerüstet. Die ausgekreiste Reaktionslösung wird in einer an sich bekannten separaten Destillationskolonne aufgearbeitet, die in der Zeichnung nicht dargestellt ist.

### Beispiele

Die nachfolgenden Beispiele 1 bis 4 wurden In einer Versuchsanlage (20-Liter-Edelstahlapparatur) durchgeführt, die analog aufgebaut ist, wie die in der Zeichnung gezeigte Anlage. Zuerst wurde (a)) der aktivierte Katalysator (4,0 kg; 3,7 % Re₂O₇ + 1,8 % B₂O₃ auf Al₂O₃) unter inerten Bedingungen In die Festbettanordnung abgefüllt und mit 70 g Me₄Sn versetzt, dann mit 4 Liter trockenem Toluen aufgefüllt und beidseitig verschlossen. Anschließend wurde (b)) 10 Liter Toluen in den Schleifenreaktor gefüllt und mit 3 bis 5 bar Stickstoff inertisiert. Der Stickstoff wurde langsam über ein Entspannungsventil abgelassen und der Reaktor mit 5 bis 6 bar (bei Einsatz von Ethen) bzw. 1,5 bar (bei Einsatz von Isobuten) mit Reaktionsgas beaufschlagt und dadurch das Inertgas ausgetauscht.

Danach wurde (c)) die mit dem Katalysator gefüllte Festbettanordnung geöffnet, die Umlaufpumpe in Betrieb gesetzt und der Reaktorinhalt mit Reaktionsgas umgepumpt, bis nach ca. 30 Minuten so gut wie keine Gasaufnahme mehr feststellbar war. Anschließend (d)) erfolgten mehrere Reaktionszyklen, durch Zudosierung von mit Toluen, verdünntem cyclischem Olefin und dem zusätzlichen Eintrag von dispergiertem Reaktionsgas (acyclisches Olefin) über die Strahlpumpe. Das Reaktionsgemisch wird unter Zuführung von Reaktionsgas durch Dispergierung solange im Kreislauf gefahren, bis keine Aufnahme an Reaktionsgas mehr erfolgt. Dieser Zeitpunkt ist erreicht, wenn der Volumenstrom an Reaktionsgas eine bestimmte Untergrenze unterschreitet, die bei Ethen 0,2 Normliter und bei Isobuten 0,3 Normliter beträgt.

Ein Reaktionszyklus ist beendet, wenn keine Gasaufnahme mehr feststellbar ist.

Nach Beendigung eines jeden Reaktionszyklus wird eine Teilmenge an Reaktionslösung ausgekreist und destillativ In Lösungsmittel, Zielprodukt, Oligomerengemisch und gegebenenfalls Destillationsrückstand getrennt. Nach dem Auskreisen einer Teilmenge an Reaktionslösung beginnt ein neuer Reaktionszyklus mit der Zudosierung von cyclischem Olefin und der Nachdosierung von Reaktionsgas.

### Beispiel 1: Ethenolyse von Cycloocten

Nach den Verfahrensschritten a) bis c) wurde der erste Reaktionszyklus mit der Dosierung einer ersten Teilmenge Cycloocten (45,12 mol = 5,5 kg; 90,4 %-ig) in 3,8 Liter Toluen begonnen. Die Dosierzeit betrug zweleinhalb Stunden, die durchschnittliche Ethen-Aufnahme lag zwischen 2,5 und 6,0 Normliter/min.

Die sich anschließende Nachreaktionsphase zur Annäherung an das chemische Gleichgewicht zwischen den Komponenten wurde bei einer Ethen-Aufnahme von 0,2 Normliter/min nach 70 Minuten abgebrochen und somit der erste Reaktionszyklus beendet. Im Anschluss daran wurden ca. 7,5 kg Reaktionslösung unter Betriebsdruck des Reaktors ausgekreist und dabei vorsichtig entspannt.

Danach erfolgte in einem weiteren Reaktionszyklus die Dosierung einer zweiten Teilmenge Cycloocten (15,75 mol = 1,92 kg; 90,4 %-ig) in 5,9 Liter Toluen und die Nachreaktion wurde bis zur Erreichung einer Ethen-Aufnahme von 0,2 Normliter/min fortgeführt. Diese Semlbatch-Fahrweise wurde solange fortgesetzt, bis die gaschromatografische Untersuchung einer ausgekreisten Probe einen Cycloocten-Umsatz von 98 % unterschritt. Dabei verlängerte sich die Gesamtreaktionszeit pro Zyklus allmählich und betrug beim letzten Durchlauf ca. 7 Stunden.

Insgesamt wurden 19 Reaktionszyklen innerhalb einer Gesamtzelt von 92,5 Stunden durchgeführt. Während dieser Zeit wurden insgesamt 328,5 mol = 40,05 kg (90,4 %-Ig) Cycloocten in 126,9 Liter Toluen (d. h. im Durchschnitt ca. 2,6 mol/l) umgesetzt. Der Verbrauch an Ethen (ohne Recycling von entspanntem Gas) betrug 8073 Normllter.

Die zeitliche Veränderung des Durchsatzes an verbrauchtem Ethen, bezogen auf den eingesetzten Katalysator, geht aus der folgenden Übersicht hervor:

| | | | | | |
|---|---|---|---|---|---|
| Zeit [min] | 100 | 250 | 500 | 1000 | 1500 |
| Durchsatz Ethen [mol*kg⁻¹*h⁻¹] | 5,03 | 2,61 | 1,71 | 1,23 | 1,05 |

Weitere Ergebnisse zum Verlauf der Reaktion sind in Tabelle 1 zusammengestellt.

Aus der destillativen Aufarbeitung der egallsierten Reaktionslösungen wurde für die einzelnen Komponenten folgende Massenbilanz ermittelt:

| | C₈ | C₁₀ | C₁₈ | C₂₆ | >=C₃₄ |
|---|---|---|---|---|---|
| Menge [kg] | 0,40 | 17,35 | 10,90 | 5,65 | 4,55 |
| Umsatz [%] | 98,7 | | | | |

Bezogen auf die Zielverbindung 1,9-Decadien erhält man als Verbrauchszahl 2,09 kg Cycloocten/kg 1,9-Decadien.

Das 1,9-Decadien ist eine farblose Flüssigkeit; Siedetemperatur: 98 - 99 °C /120 mbar; Brechungsindex bei 20°C / 589 nm: 1,4325; Dichte bei 20 °C: 0,7540 g/cm³; Gehalt ca. 98%.

### Beispiel 2: Ethenolyse von 1,9,17-Octadecatrien (C₁₈-Trien)

Nach den Verfahrensschritten a) bis c) wurde der erste Reaktionszyklus mit der Dosierung einer ersten Teilmenge des C₁₈-Triens (9,86 mol = 2,5 kg; 2 Isomere, ca. 98 %-ig) in 4,6 Liter Toluen begonnen. Die Dosierung erfolgte innerhalb von 75 Minuten, wobei die durchschnittliche Ethen-Aufnahme 2 bis 4 Normliter/min betrug. Die sich anschließende Nachreaktionsphase zur weitgehenden Einstellung des chemischen Gleichgewichtes zwischen den Komponenten wurde bei Erreichen eines Ethen-Verbrauches von 0,7 Normliter/min abgebrochen und danach 3,3 kg Reaktionslösung ausgekreist.

Im Anschluss daran erfolgte In einem zweiten Reaktionszyklus die Dosierung einer weiteren 2,5 kg Teilmenge C₁₈-Trien, gelöst in 4,6 Liter Toluen. Nach Durchlauf der zweiten Teilmenge wurde der Versuch abgebrochen, obwohl die Reaktivität des Katalysatorsystems für weitere Reaktionszyklen noch ausreichend war.

Insgesamt wurden 4,9 kg C₁₈-Trien In 18,5 Liter Toluen (d. h. im Durchschnitt ca. 1,1 mol/l) eingesetzt. Der Verbrauch an Ethen betrug (ohne Recycling) ca. 439 Normilter. Weitere Ergebnisse zum Verlauf der Reaktion sind in Tabelle 1 zusammengefasst.

Aus der destillativen Aufarbeitung der egallsierten Reaktionslösungen wurde für die einzelnen Komponenten folgende Massenbilanz erstellt:

| | C₈ | C₁₀ | C₁₈ | C₂₆ | >=C₃₄ |
|---|---|---|---|---|---|
| Menge [kg] | 0,03 | 4,01 | 2,19 | 0,09 | 0 |
| Umsatz [%] | | | 63,0 | | |

Bezogen auf die Zielverbindung 1,9-Decadien erhält man als Verbrauchszahl 1,22 kg C₁₈-Trien/kg 1,9-Decadien. Nach Abzug des unumgesetzten C₁₈-Triens, das nach destillativer Abtrennung noch einmal eingesetzt werden kann, ist dieser Wert 0,68.

### Vergleichsbeispiel: Ethenolyse von Cycloocten

Es wurde in analoger Weise wie im Beispiel 1 verfahren, nur mit dem Unterschied, dass die Strahlpumpe der Anlage ausgebaut und durch ein Rohrstück ersetzt wurde, in das die Zuleitung für das Inert- bzw. Reaktionsgas eingebunden ist.

Im Schleifenreaktor befand sich somit nur im Lösungsmittel gelöstes Reaktionsgas (Ethen).

Nach den Verfahrensschritten a) bis c) wurde der erste Reaktionszyklus mit der Dosierung einer ersten Teilmenge Cycloocten (30,8 mol = 3,75 kg; 90,4 %-ig) in 16 Liter Toluen begonnen. Die Dosierzeit betrug 65 Minuten, die durchschnittliche Ethen-Aufnahme lag zwischen 4 und 5 Normliter/min. Die sich anschließende Nachreaktionsphase zur Annäherung an das chemische Gleichgewicht zwischen den Komponenten wurde nach 3,2 Stunden bei einer Ethen-Aufnahme von 0,25 Norm-Ilter/min abgebrochen.

Anschließend wurden ca. 7,5 kg Reaktionslösung unter Betriebsdruck des Reaktors ausgekreist und dabei vorsichtig entspannt.

Danach erfolgte ein weiterer Reaktionszyklus durch Dosierung einer zweiten in Toluen gelösten Teilmenge Cycloocten (analog wie im ersten Reaktionszyklus). Diese Semibatch-Fahrweise wurde über weitere 7 Reaktionszyklen und einer Gesamtdauer von 31,75 Stunden aufrechterhalten.

Insgesamt wurden dabei 13,42 kg Cycloocten in 57,5 Liter Toluen (d. h. im Durchschnitt ca. 2,1 mol/l) umgesetzt. Der Verbrauch an Ethen (ohne Recycling) von entspanntem Gas) betrug ca. 2070 Normliter.

Die zeitliche Veränderung des Durchsatzes an verbrauchtem Ethen, bezogen auf den eingesetzten Katalysator, geht aus der folgenden Übersicht hervor:

| | | | | | |
|---|---|---|---|---|---|
| Zeit [min] | 100 | 250 | 500 | 1000 | 1500 |
| Durchsatz Ethen [mol*kg⁻¹*h⁻¹] | 2,52 | 1,36 | 0,80 | 0,67 | 0,66 |

Es wurde auf eine destillative Aufarbeitung der Reaktionslösung verzichtet.

Weitere Ergebnisse zum Reaktionsverlauf sind in der nachfolgenden Tabelle 1 angegeben.

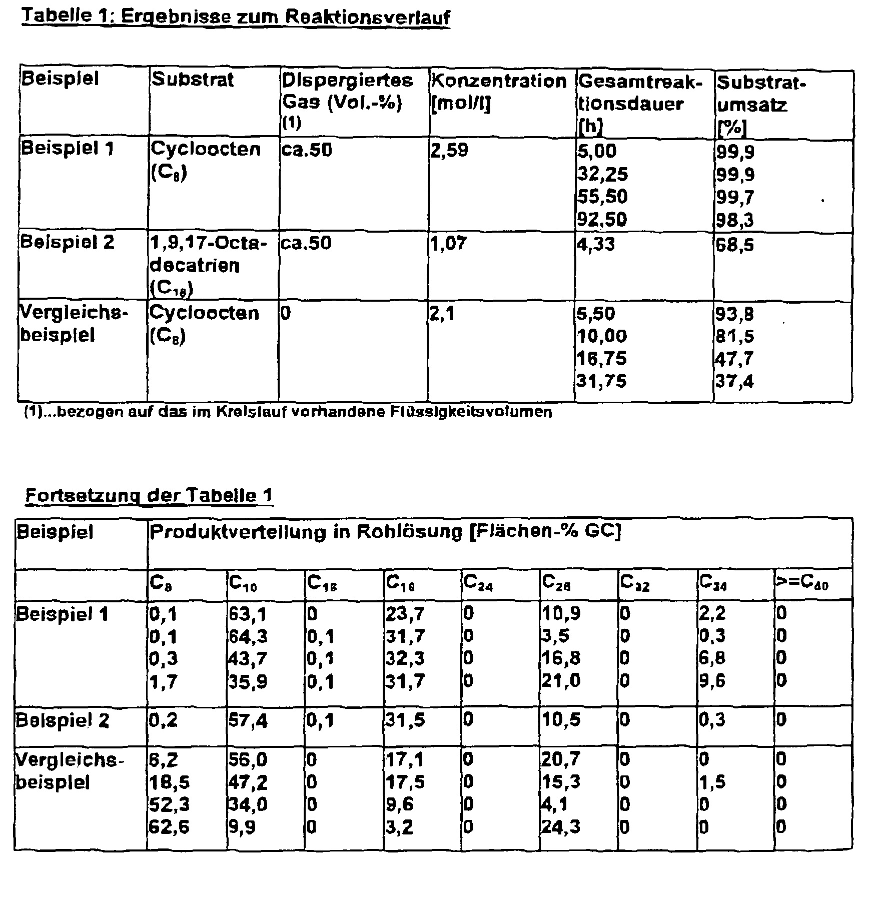

Ein Vergleich zwischen Beispiel 1 und dem Vergleichsbeispiel zeigt, dass durch den zusätzlichen Eintrag von Reaktionsgas in dispergierter Form über die Strahlpumpe während der gesamten Reaktionsdauer der Verbrauch an Ethen (Reaktionsgas) um ca. 50% höher liegt, als bei einer Fahrweise mit nur im Lösungsmittel gelöstem Ethen.

Wie die Ergebnisse zeigen wird bei der Verfahrenweise gemäß Beispiel 1 der Anteil an höhermolekularen Verbindungen in der Reaktionslösung verringert und die Ausbeute an Zielprodukt erhöht. Bei dem Vergleichsbeispiel ist der Umsatz an Cycloocten deutlich geringer und fällt sehr schnell ab, wie die Ergebnisse der Tabelle 1 belegen. Bereits nach kurzer Zeit ist bei einer Fahrweise ohne Strahlpumpe (nur bei Vorliegen von im Lösungsmittel gelöstem Reaktionsgas) die Bildung von höhermolekularen Reaktionsprodukten (insbesondere das 1,9,17,25-Hexacosatetraen, C₂₆-Tetraen) bevorzugt.

### Beispiel 3: Ethenolyse eines Gemisches aus Cycloocten und Oligomere

Nach den Verfahrensschritten a) bis c) wurde der erste Reaktionszyklus mit der Dosierung einer ersten Teilmenge eines Gemisches aus 14,98 kg Cycloocten (90,4 %-ig) und 21,10 kg Oligomere (C₁₈-Trien, C₂₆-Tetraen, C₃₄-Pentaen, erhalten nach der Destillation ausgekreister Reaktionslösung gemäß Beispiel 1) in 125 Liter Toluen gelöst, begonnen.

Die analog durchgeführte semikontinuierliche Ethenolyse dieses Gemisches lieferte nach Abtrennung des Lösungsmittels 37,20 kg Reaktionsprodukt. Der Verbrauch an Ethen (ohne Recycling von entspanntem Gas) betrug 3020 Normliter.

Die Destillation des Reaktionsproduktes ergab für die einzelnen Komponenten folgende Massenbilanz:

| | C₈ | C₁₀ | C₁₈ | C₂₆ | >=C₃₄ |
|---|---|---|---|---|---|
| Menge [kg] | 0,30 | 16,70 | 10,53 | 5,36 | 4,31 |

Bezogen auf die Zielverbindung 1,9-Decadien erhält man als Verbrauchszahl 0,81 kg Cycloocten/kg 1,9-Decadien.

### Beispiel 4: Isobutenolyse von Cyclopenten

Es wurde analog wie in Beispiel 1 verfahren, nur dass anstatt von Ethen Isobuten und anstatt Cycloocten Cyclopenten eingesetzt wurden.

Nach Beaufschlagung des Reaktorinhaltes mit isobuten bei 1,5 bar wurde im ersten Reaktionszyklus eine erste Teilmenge Cyclopenten (36,0 mol = 2,49 kg; 98,5 %-ig) in 3,2 Liter Toluen gelöst innerhalb von zwei Stunden zudosiert. Anschließend erfolgte eine entsprechende Nachreaktionsphase bis der gemessene Isobuten-Verbrauch etwa 0,3 Normliter/min erreicht hatte. Danach wurde der Reaktionszyklus abgebrochen, ca. 6 kg Reaktionslösung unter Betriebsdruck ausgekreist und dabei vorsichtig entspannt.

Im Anschluss daran erfolgte in einem weiteren Reaktionszyklus die Dosierung der zweiten Menge Cyclopenten (12,0 mol = 0,83 kg; 98,5 %-ig) in 4,8 Liter Toluen. Die Reaktion wurde analog wie im ersten Reaktionszyklus weitergeführt. Diese Semibatch-Fahrweise wurde solange fortgesetzt, bis die gaschromatografische Untersuchung einer ausgekreisten Probe einen Cyclopenten-Umsatz von 96 % unterschritt.

Nach 19 weiteren Reaktionszyklen und einer Gesamtdauer von 84 Stunden wurde die Reaktion abgebrochen. Während dieser Zeit wurden insgesamt 18,8 kg (276,35 mol) Cyclopenten in 110 Liter Toluen umgesetzt (d. h. im Durchschnitt 2,5 mol/l). Der Verbrauch an Isobuten (ohne Recycling von entspanntem Gas) betrug 6809,4 Normliter.

Die Ergebnisse zum Verlauf der Reaktion sind in Tabelle 2 aufgeführt.

Aus der destillativen Aufarbeitung der egalisierten Reaktionslösung wurde für die einzelnen Komponenten folgende Massenbilanz erstellt (geringfügige Mengen an C₁₀- und C₁₁-Verbindungen wurden nicht berücksichtigt):

| | C₅ | C₉ | C₁₄ | C₁₉ | C₂₄ | C₂₉ |
|---|---|---|---|---|---|---|
| Menge [kg] | 0,66 | 18,44 | 9,82 | 4,15 | 1,20 | 0 |
| Umsatz [%] | 98,1 | | | | | |

Bezogen auf die Zielverbindung 7-Methyl-1,6-octadien erhält man als Verbrauchszahl 1,02 kg Cyclopenten / kg 7-Methyl-1,6-octadien.

Auch hier lässt sich die Ausbeute an Zielprodukt verbessern, da bevorzugt die niedermolekularen Oligomeren entstehen, die nach entsprechender Aufarbeitung erneut eingesetzt werden können.

Das 7-Methyl-1,6-octadien ist eine farblose Flüssigkeit; Siedetemperatur: 77 - 79 °C /120 mbar; Brechungsindex bei 20 °C / 589 nm: 1,4355; Dichte bei 20 °C: 0,7505; Gehalt ca. 98 %.

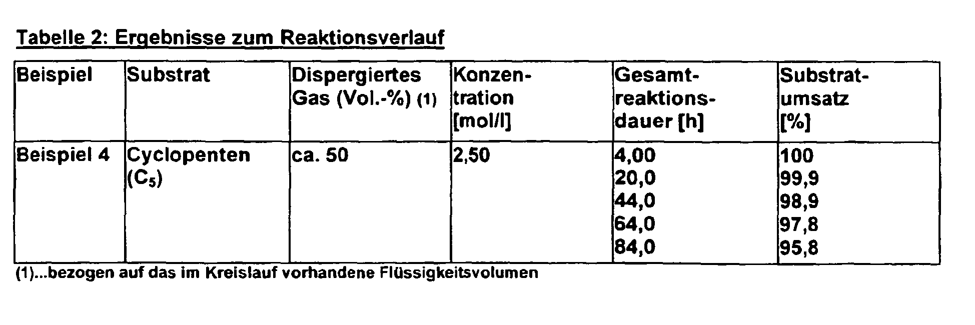

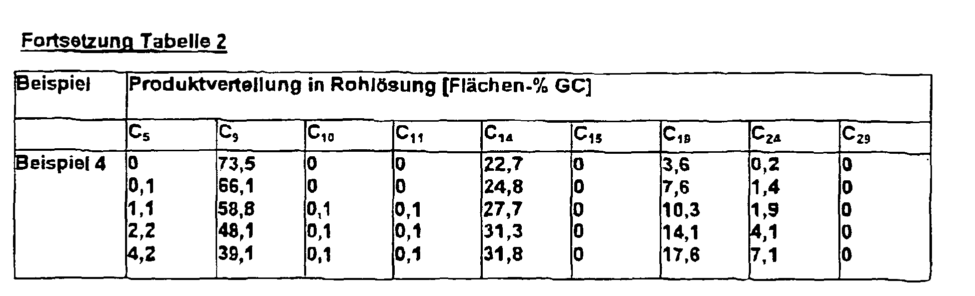

## Patentansprüche

1. Verfahren zur Durchführung einer ringöffnenden Kreuzmetathesereaktion zwischen einem flüssigen cyclischen Olefin und einem acyclischen Olefin als Reaktionsgas an einem metathetisch aktiven Katalysatorsystem als Festbett, bei dem das cyclische Olefin in einem geeigneten Lösungsmittel gelöst wird, das Reaktionsgas unter Überdruck in einem metathetisch inerten Lösungsmittel gelöst wird und das Gemisch der beiden Reaktionskomponenten im Kreislauf über den Festbettkatalysator gepumpt und Reaktionsgas nachdosiert wird, **dadurch gekennzeichnet, dass** während der Reaktionszyklen zusätzlich in das Reaktionsgemisch noch Reaktionsgas in einer Menge von mindestens 25 Vol.-%, bezogen auf das im Kreislauf vorhandene Flüssigkeitsvolumen, feinblasig dispergiert wird, unter Bildung von Blasen mit einem Durchmesser von 0,1 bis 1 mm, wobei das Reaktionsgemisch verwirbelt wird, und in unmittelbarer Nähe des Festbettkatalysators dispergiertes Reaktionsgas in dem Maße nachgelöst wird, wie gelöstes Reaktionsgas verbraucht und **dadurch** die Reaktionskomponenten am Katalysator in überwiegendem Maße zu niedermolekularen Verbindungen (Zielprodukt) umgesetzt werden, wobei als Nebenprodukte Oligomere mit bis zu drei Cycloolefineinheiten entstehen, die nach separater Abtrennung aus der Reaktionslösung wieder in den Kreislauf zurückgeführt werden.

2. Verfahren nach Anspruch 1, **dadurch gekennzeichnet, dass** die Reaktion in einem Schleifenreaktor mit integriertem Festbettkatalysator erfolgt, wobei zur Dispergierung des Reaktionsgases dieses über eine in den Schleifenreaktor eingebundene Strahlpumpe eingetragen wird.

3. Verfahren nach einem der Ansprüche 1 oder 2, **dadurch gekennzeichnet, dass** vor der Zuführung des cyclischen Olefins das Katalysatorsystem unter inerten Bedingungen in die Festbetteinrichtung eingetragen wird, diese anschließend mit einem metathetisch inerten Lösungsmittel aufgefüllt und die Festbetteinrichtung dicht verschlossen wird.

4. Verfahren nach einem der Ansprüche 1 bis 3, **dadurch gekennzeichnet, dass** während des geschlossenen Zustandes der Festbetteinrichtung in den Schleifenreaktor die erforderlichen Menge an metathetisch inertem Lösungsmittel gegeben wird, Inertgas eingeleitet und danach Inertgas durch unter Überdruck zugeführtes Reaktionsgas ausgetauscht wird.

5. Verfahren nach einem der Ansprüche 1 bis 4, **dadurch gekennzeichnet, dass** nach erfolgtem Austausch von Inertgas durch Reaktionsgas die Festbetteinrichtung geöffnet und das Gemisch Lösungsmittel/Reaktlonsgas unter Zuführung von Rektionsgas so lange im Kreislauf gefahren wird, bis keine Gasaufnahme mehr erfolgt.

6. Verfahren nach einem der Ansprüche 1 bis 5, **dadurch gekennzeichnet, dass** nach Beendigung der Gasaufnahme im Lösungsmittel ein erster Reaktionszyklus beginnt, cyclisches Olefin zudosiert wird und solange im Kreislauf gefahren wird, bis keine Aufnahme an Reaktionsgas mehr erfolgt und damit dieser Reaktionszyklus beendet wird.

7. Verfahren nach einem der Ansprüche 1 bis 6, **dadurch gekennzeichnet, dass** unmittelbar nach Beendigung eines Reaktionszyklus eine Teilmenge an Reaktionslösung ausgekreist und destillativ in Lösungsmittel, Zielprodukt, Oligomerengemisch und Destillationsrückstand getrennt wird.

8. Verfahren nach einem der Ansprüche 1 bis 7, **dadurch gekennzeichnet, dass** nach erfolgtem Auskreisen der Reaktionslösung ein weiterer Reaktionszyklus beginnt, entweder durch Zudosierung von in Lösungsmittel gelöstem cycllschen Olefin oder gelösten Oligomeren oder eines Gemisches bestehend aus cyclischem Olefin und Oligomeren.

9. Verfahren nach einem der Ansprüche 1 bis 8, **dadurch gekennzeichnet, dass** cyclisches Olefin in Konzentrationen von 0,1 bis 5 Mol/l eingesetzt wird.

10. Verfahren nach einem der Ansprüche 1 bis 9, **dadurch gekennzeichnet, dass** das cyclische Olefin aus der Gruppe der Verbindungen Cyclopenten, Cyclohepten, Cycloocten, Cyclooctadien, 1,5-Dimethyl-1,5-cyclooctadien, Cyclodecen, Cyclododecen, 1,9-Cyclohexadecadien, 1,9,17-Tetracosatrien, Alpha-Pinen, Caren, Limonen, Norbornen und Norbornadien ausgewählt wird.

11. Verfahren nach einem der Ansprüche 1 bis 10, **dadurch gekennzeichnet, dass** das Reaktionsgas aus der Gruppe der Verbindungen Ethen, Propen, Buten-1, Buten-2 und isobuten ausgewählt wird.

12. Verfahren nach einem der Ansprüche 1 bis 11, **dadurch gekennzeichnet, dass** dieses bei Temperaturen oberhalb der Siedetemperatur des acyclischen Olefins durchgeführt, wobei das Reaktionsgas aus der Gruppe der Verbindungen 1-Penten, 2-Penten, 2-Methyl-2-buten (Isoamylen), 1-Hexen, 2-Hexen, 3-Hexen, 2-Methyl-1-penten, 3-Methyl-1-penten, 4-Methyl-1-penten (Isohexen), ausgewählt wird.

13. Anlage zur Durchführung des Verfahren nach mindestens einem der vorhergehenden Ansprüche, **dadurch gekennzeichnet, dass** diese aus einem Schleifenreaktor (SR) besteht, in dessen Kreislaufleitung (L) eine absperrbare Festbettkatalysatoreinrichtung (1), ein Ausdehnungsgefäß (2), eine Strahlpumpe (3) und eine Kreislaufpumpe (4) eingebunden sind, wobei das Ausdehnungsgefäß (2) in Strömungsrichtung vor der Kreislaufpumpe (4), die Festbetteinrichtung (1) nach der Kreislaufpumpe (4), zwischen der Kreislaufpumpe (4) und der Strahlpumpe (3), angeordnet sind, die Strahlpumpe (3) mit dem Ausdehnungsgefäße (2) in Verbindung steht, wobei der Saugstutzen (3a) der Strahlpumpe (3) über eine Leitung (5) mit dem Ausdehnungsgefäß (2) verbunden ist und der Diffusor (3b) der Strahlpumpe (3) in das Ausdehnungsgefäß (2) ragt, und in die Kreislaufleitung (L), vor der Strahlpumpe (3), eine erste absperrbare Zuführungsleitung (6) für cyclisches Olefin oder Lösungsmittel und eine zweite absperrbare Zuführungsleitung (7) für das Reaktions-/Inertgas sowie nach der Festbetteinrichtung (1) eine absperrbare Leitung (8) zur Auskreisung der Reaktionslösung eingebunden sind.

14. Anlage nach Anspruch 13, **dadurch gekennzeichnet, dass** die Zuführungsleitung für das Reaktions-/Inertgas in die Leitung (7) eingebunden ist, die den Saugstutzen (3a) der Strahlpumpe (3) mit dem Ausdehnungsgefäß (2) verbindet, wobei die Zuführungsleitung (7) vor der Einbindungsstelle (9) mittels eines Ventils (10) absperrbar ist.

15. Anlage nach Anspruch 13, **dadurch gekennzeichnet, dass** die Zuführungsleitung (7) für das Reaktions-/Inertgas unmittelbar oberhalb der Strahlpumpe (3) in die Kreislaufleitung (L) eingebunden ist.

16. Anlage nach einem der Ansprüche 13 bis 15, **dadurch gekennzeichnet, dass** mindestens eine Festbetteinrichtung (2) in vertikaler Richtung angeordnet ist.

17. Anlage nach einem der Ansprüche 13 bis 16, **dadurch gekennzeichnet, dass** in der Kreislaufleitung (L) vor und nach der Festbetteinrichtung (1) jeweils ein Absperrventil (11, 12) eingebunden ist.

## Claims

1. Process for carrying out a ring opening cross-metathesis reaction between a liquid cyclic olefin and an acyclic olefin as a reactive gas on a metathetically active catalyst system as a fixed bed, in which the cyclic olefin is dissolved in a suitable solvent, the reactive gas is dissolved under higher pressure in a metathetically inert solvent and the mixture of the two reaction components in the circuit is pumped over the fixed bed catalyst and reactive gas is added, **characterised in that** additional reactive gas, in quantities of at least 25% of the volume of the liquid present in the circuit, is dispersed into the reaction mixture during the reaction cycles in the form of fine bubbles, forming bubbles with a diameter of 0.1 to 1 mm, whereby the reaction mixture is swirled and dispersed reactive gas in the immediate vicinity of the fixed bed catalyst is redissolved in the same proportion as the dissolved reactive gas is consumed and thereby the reaction components on the catalyst are largely converted to low-molecular compounds (target product), with oligomers with up to three cyclo olefin units being produced as by-products, which are recirculated after separate separation from the reaction solution.

2. Process according to claim1, **characterised in that** the reaction takes place in a loop reactor with an integrated fixed bed catalyst, with the reactive gas fed in by means of a jet pump integrated into the loop reactor to disperse said reactive gas.

3. Process according to one of the claims 1 or 2, **characterised in that** the catalyst system is inserted into the fixed bed device under inert conditions before the feeding in of the cyclic olefin and said fixed bed device is then filled with a metathetically inert solvent and the fixed bed device is tightly sealed.

4. Process according to one of the claims 1 to 3, **characterised in that** the required quantity of metathetically inert solvent is added into the loop reactor during the closed condition of the fixed bed device, inert gas is fed in which is subsequently replaced by reactive gas fed in under higher pressure.

5. Process according to one of the claims 1 to 4, **characterised in that** after the inert gas has been replaced by the reactive gas, the fixed bed device is opened and the mixture of solvent/reactive gas is circulated, with reactive gas being fed in, until no further gas absorption takes place.

6. Process according to one of the claims 1 to 5 **characterised in that** an initial reaction cycle commences after gas absorption in the solvent has ended, cyclic olefin is dosed in and circulated in the circuit until no more reactive gas is absorbed, whereupon this reaction cycle ends.

7. Process according to one of the claims 1 to 6, **characterised in that** immediately after the ending of a reaction cycle, a portion of the reaction solution is removed from the circuit and separated by distillation into solvent, target product, oligomer mixture and distillation residue.

8. Process according to one of the claims 1 to 7, **characterised in that** a further reaction cycle commences after the reaction solution has been removed from the circuit, either through dosing in of the cyclic olefin dissolved in the solvent or the dissolved oligomers or a mixture consisting of cyclic olefin and oligomers.

9. Process according to one of the claims 1 to 8, **characterised in that** cyclic olefin is used at concentrations of 0.1 to 5 mol/l.

10. Process according to one of the claims 1 to 9, **characterised in that** the cyclic olefin is selected from the group of compounds cyclopentenes, cycloheptenes, cyclooctenes, cyclooctadienes, 1.5-dimethyl-1.5-cyclooctadiene, cyclodecenes, cyclododecenes, 1.9 cyclohexadecadiene, 1.9.17-tetracosatriene, alpha-pinenes, careens, limonenes, norbornenes and norbornadienes.

11. Process according to one of the claims 1 to 10, **characterised in that** the reactive gas is selected from the group of compounds ethylene, propylene, butene-1, butene-2 and isobutylene.

12. Process according to one of the claims 1 to 11, **characterised in that** the process is carried out at temperatures above the boiling temperature of the acyclic olefin, with the reactive gas selected from the group of compounds 1-pentene, 2-pentene, 2-methyl-2-butene (isoamylene), 1-hexene, 2-hexene, 3-hexene, 2-methyl-1-pentene, 3-methyl-1-pentene, 4-methyl-1-pentene (isohexene).

13. Plant for carrying out the process according to at least of one of the foregoing claims, **characterised in that** this plant consists of a loop reactor (SR), in whose circulation line (L) an fixed bed catalyst device (1) that can be shut off, an expansion vessel (2), a jet pump (3) and a circulation pump (4) are integrated, whereby in the flow direction the expansion vessel (2) is positioned in front of the circulation pump (4), the fixed bed device (1) is positioned after the circulation pump (4), between the circulation pump (4) and the jet pump (3), the jet pump (3) is connected to the expansion vessel (2), whereby the intake from side arm (3a) of the jet pump (3) is connected via a line (5) to the expansion vessel (2) and the diffuser (3b) of the jet pump (3) protrudes into the expansion vessel (2) and an initial feed line (6) for cyclic olefin or solvent that can be shut off and a second feed line (7) for the reactive/inert gas that can be shut off are integrated into the circulation line (L) in front of the jet pump (3) and a line (8) that can be shut off for removing the reaction solution from the circuit is integrated into said circulation line (L) after the fixed bed device (1).

14. Plant according to claim 13, **characterised in that** the feed line for the reactive/inert gas is integrated into the line (7) connecting the intake from side arm (3a) of the jet pump (3) to the expansion vessel (2), whereby the feed line (7) in front of the jointing location (9) can be shut off by means of a valve (10).

15. Plant according to claim 13, **characterised in that** the feed line (7) for the reactive/inert gas is integrated into the circulation line (L) directly above the jet pump (3).

16. Plant according to one of the claims 13 to 15, **characterised in that** at least one fixed bed device (2) is positioned vertically.

17. Plant according to one of the claims 13 to 16, **characterised in that** shut off valves (11, 12) are integrated in the circulation line (L) before and after the fixed bed device (1).

## Revendications

1. Procédé pour la réalisation d'une réaction d'ouverture de cycle par métathèse croisée entre une oléfine cyclique liquide et une oléfine acyclique se présentant sous forme de gaz de réaction sur un système catalyseur de métathèse active se présentant sous forme de lit fixe, pour lequel l'oléfine cyclique est dissoute dans un solvant approprié, le gaz de réaction est dissous sous surpression dans un solvant de métathèse inerte et le mélange des deux composants de la réaction est pompé dans le circuit sur le catalyseur en lit fixe, un redosage du gaz de réaction étant effectué, **caractérisé en ce que**, pendant les cycles de réaction, une quantité supplémentaire de gaz de réaction d'au moins 25 % vol. par rapport au volume de liquide présent dans le circuit est dispersée en plus en fines bulles dans le mélange de la réaction, en formant des bulles d'un diamètre compris entre 0,1 et 1 mm, le mélange de la réaction tourbillonnant, et le gaz de réaction dispersé directement à côté du catalyseur en lit fixe est de nouveau dissous en proportion de la consommation de gaz de réaction dissous, ce qui transforme les composants de la réaction dans le catalyseur principalement en proportion des liaisons de faible poids moléculaire (produit d'arrivée), ce qui donne sous forme de sous-produits des oligomères présentant jusqu'à trois unités de cyclooléfines qui sont ramenés dans le circuit après avoir été évacués séparément de la solution de la réaction.

2. Procédé selon la revendication 1, **caractérisé en ce que** la réaction s'effectue dans une colonne à bulles à écoulement en boucle muni d'un catalyseur en lit fixe intégré, le gaz de réaction étant introduit en vue de sa dispersion par une pompe à jet insérée dans la colonne à bulles à écoulement en boucle.

3. Procédé selon l'une quelconque des revendications 1 ou 2, **caractérisé en ce qu'**avant l'introduction de l'oléfine cyclique, le système catalyseur est introduit dans des conditions inertes dans le dispositif en lit fixe, qui est ensuite rempli de solvant de métathèse inerte, puis le dispositif en lit fixe est fermé hermétiquement.

4. Procédé selon l'une quelconque des revendications 1 à 3, **caractérisé en ce que**, pendant la fermeture du dispositif en lit fixe, la quantité nécessaire de solvant de métathèse inerte est introduite dans la colonne à bulles à écoulement en boucle, du gaz inerte y étant introduit et ensuite, le gaz inerte est échangé contre le gaz de réaction amené sous surpression.

5. Procédé selon l'une quelconque des revendications 1 à 4, **caractérisé en ce qu'**une fois le gaz inerte échangé contre le gaz de réaction, le dispositif en lit fixe est ouvert et le mélange de solvant/gaz de réaction circule en boucle dans le système en amenant du gaz de réaction jusqu'au terme de l'absorption du gaz.

6. Procédé selon l'une quelconque des revendications 1 à 5, **caractérisé en ce qu'**au terme de l'absorption de gaz dans le solvant, un premier cycle de réaction commence, une quantité d'oléfine cyclique est rajoutée et le mélange circule en boucle dans le système jusqu'au terme de toute absorption du gaz de réaction, mettant ainsi un terme à ce cycle de réaction.

7. Procédé selon l'une quelconque des revendications 1 à 6, **caractérisé en ce que**, directement à la fin d'un cycle de réaction, une quantité partielle de solution de la réaction est évacuée du circuit et séparée par distillation en solvant, produit d'arrivée, mélange d'oligomères et résidu de distillation.

8. Procédé selon l'une quelconque des revendications 1 à 7, **caractérisé en ce qu'**au terme de l'évacuation hors du circuit de la solution de la réaction, un autre cycle de réaction commence, soit par ajout d'oléfine cyclique dissoute dans un solvant ou d'oligomères dissous ou d'un mélange se composant d'oléfine cyclique et d'oligomères.

9. Procédé selon l'une quelconque des revendications 1 à 8, **caractérisé en ce que** l'oléfine cyclique est utilisée dans des concentrations comprises entre 0,1 et 5 mol/l.

10. Procédé selon l'une quelconque des revendications 1 à 9, **caractérisé en ce que** l'oléfine cyclique est sélectionnée parmi le groupe de liaison des cyclopentènes, cycloheptènes, cyclooctènes, cyclooctadiènes, 1,5-diméthyle-1,5-cyclooctadiènes, cyclodécènes, cyclododécènes, 1,9-cyclohexadécadiènes, 1,9,17-tétracosatriènes, alphapinènes, carènes, limonènes, norbornènes et norbornadiènes.

11. Procédé selon l'une quelconque des revendications 1 à 10, **caractérisé en ce que** le gaz de réaction est sélectionné parmi le groupe de liaison des éthènes, propènes, butènes-1, butènes-2 et isobutènes.

12. Procédé selon l'une quelconque des revendications 1 à 11, **caractérisé en ce que** celui-ci est réalisé à des températures supérieures à la température d'ébullition de l'oléfine cyclique, le gaz de réaction étant sélectionné parmi le groupe de liaison des 1-pentènes, 2-pentènes, 2-méthyl-2-butènes (isoamylènes), 1-hexènes, 2-hexènes, 3-hexènes, 2-méthyl-1-pentènes, 3-méthyt-1-pentènes, 4-méthyl-1-pentènes (isohexènes).

13. Installation pour la mise en oeuvre du procédé selon au moins l'une quelconque des revendications précédentes, **caractérisée en ce que** celle-ci se compose d'une colonne à bulles à écoulement en boucle (SR) dans la conduite de circuit (L) de laquelle sont insérés un dispositif à catalyseur en lit fixe (1) pouvant être fermé, un vase d'expansion (2), une pompe à jet (3) et une pompe de recyclage (4), le vase d'expansion (2) étant disposé dans le sens du courant avant la pompe de recyclage (4), le dispositif en lit fixe (1) étant disposé après la pompe de recyclage (4) entre la pompe de recyclage (4) et la pompe à jet (3), la pompe à jet (3) étant reliée au vase d'expansion (2), la buse d'aspiration (3a) de la pompe à jet (3) étant reliée au vase d'expansion (2) par l'intermédiaire d'une conduite (5) et le diffuseur (3b) de la pompe à jet (3) pénétrant à l'intérieur du vase d'expansion (2), et la conduite de circuit (L) comprenant avant la pompe à jet (3) une première conduite d'arrivée (6) de l'oléfine cyclique ou de solvant, pouvant être fermée, et une seconde conduite d'arrivée (7) du gaz de réaction/gaz inerte, pouvant être fermée, ainsi qu'après le dispositif en lit fixe (1) une conduite (8) pouvant être fermée pour évacuer du circuit la solution de la réaction.

14. Installation selon la revendication 13, **caractérisée en ce que** la conduite d'arrivée du gaz de réaction/gaz inerte est insérée dans la conduite (7) qui relie la buse d'aspiration (3a) de la pompe à jet (3) au vase d'expansion (2), la conduite d'arrivée (7) pouvant être fermée avant le point d'insertion (9) au moyen d'une vanne (10).

15. Installation selon la revendication 13, **caractérisée en ce que** la conduite d'arrivée (7) du gaz de réaction/gaz inerte est insérée dans la conduite de circuit (L) directement au-dessus de la pompe à jet (3).

16. Installation selon l'une quelconque des revendications 13 à 15, **caractérisée en ce qu'**au moins un dispositif en lit fixe (2) est disposé dans le sens vertical.

17. Installation selon l'une quelconque des revendications 13 à 16, **caractérisée en ce qu'**une vanne d'arrêt (11, 12) est insérée dans la conduite de circuit (L) respectivement avant et après le dispositif en lit fixe (1).
